# EUROPEAN PATENT SPECIFICATION

(11) **EP 0 853 923 B1**
(45) Date of publication and mention of the grant of the patent: **26.03.2003**
(21) Application number: 97122921.6
(22) Date of filing: 28.12.1997
(51) Int. Cl.: A61B 17/74, A61B 17/17

(54) **Intramedullary cavity nail for the treatment of fractures of the hip**
Markraumnagel für die Versorgung von Frakturen der Hüfte
Clou de cavité intramedullaire pour la traitement des fractures de la hanche

(30) Priority: 21.01.1997 IT VR970003
(43) Date of publication of application: 22.07.1998
(73) Proprietor: ORTHOFIX S.r.l., 37012 Bussolengo (VR) (IT)
(72) Inventor: Faccioli, Giovanni, 46040 Monzambano, Mantova (IT); Venturini, Daniele, 37065 Povegliano Veronese, Verona (IT); Lavini, Franco, 37121 Verona (IT); Renzi, Brivio Lodovico, 37131 Verona (IT); Ten Veldhujs, Sander, 37138 Verona (IT)
(74) Representative: Botti, Mario

(56) References cited:
- EP-A- 0 355 411
- EP-A- 0 640 318
- EP-A- 0 736 286
- FR-A- 2 713 914
- US-A- 4 622 959
- US-A- 4 875 474
- US-A- 5 429 640
- US-A- 5 562 666
- US-A- 5 573 536

## Description

### Technical Field

The present invention relates to an intramedullary cavity nail for the treatment of proximal femoral fractures of the type comprising a solid elongated body having a proximal portion with at least one hole for accommodating corresponding screws for stabilisation of the femoral neck, united to a distal portion with at least one distal hole for accommodating at least one diaphysis screw for stabilisation of the distal part of the femur.

The invention also concerns an instrument for the installation of the nail in the medullary canal of the femur.

### Background Art

It is known that pertrochanteric fractures are the most frequent in connection with those of the region of the neck of the bone amongst geriatric patients. In fact, the advanced age and the pathologies which are encountered in these patients make necessary a timely stabilisation of skeletal injuries in order to reduce to a minimum the bed confinement and the rehabilitation times by means of interventions which are less sanguinary and invasive as possible. In fact, it is necessary to avoid the complications brought about by immobilisation syndrome which may be lethal for patients in delicate metabolical compensation and it is necessary to reduce blood losses related to the intervention to a minimum.

At the same time, the syntheses means utilised must be stable in order to allow the patient to very timely assume the seated position and already after two or three days after the intervention to reassume the erect stature with progressive weight.

A known technique for the consolidation of intertrochanteric, pertrochanteric, and subtrochanteric fractures of the femur involves the use of tubular intramedullary cavity nails with a proximal portion bent by some degrees in a medium-lateral plane with respect to the smaller diameter diaphysis portion in order to adapt to the physiological curvature of the femur. Sometimes, the curvature is present in two mutually orthogonal planes in order to favour further the adaptation of the medullary cavity of the bone.

The known tubular nails are generally fixed by means of two diaphysis nails in order to block the rotation of the nail and one or two cephalic screws for compressing the spongy tissue in the focus fracture.

A first drawback of the known intramedullary cavity nails of the above described type is constituted by the fact that they have both a proximal part and a distal part of relatively elevated diameter, generally greater than 10mm, and therefore their installation requires complex drilling of the bone for the entire length of the nail and can provoke internal stresses of the bone.

Moreover, the diaphysis screws are rather close to the distal end of the nail and can provoke stress concentrations and rupture of the femur at the base of the nail.

Finally, the cephalic screws are normally mutually parallel and therefore the head can be subject to slipping with consequent losses of stability of the fracture.

EP-A-O73628 disclosed an intramedullary nail of the above described type, wherein the distal part has a diameter which is less than that of the proximal part and wherein the proximal part has a hole for accommodating a diaphysis screw.

### Disclosure of the invention:

A principal aim of the present invention is to eliminate or at least reduce the above described drawbacks by providing an intramedullary cavity nail which has characteristics of elevated robustness, reliability and biocompatibility, and which is hardly invasive.

A particular aim is to provide an intramedullary cavity nail which requires minimum bone drilling operations and skin incisions so as to limit blood loss and operating intervention and rehabilitation times.

A further aim is to provide an intramedullary cavity nail of elevated biocompatibility and of reduced cost.

Another further aim is to provide an intramedullary cavity nail which is easily installable in the medullary canal with an extremely simplified and effective instrument.

In accordance with the invention, there is provided an intramedullary cavity nail of the type defined in appended claim 1.

### Brief description of drawings

The particular characteristics and advantages of the invention will become apparent from the description of some preferred but not exclusive embodiments of the intramedullary cavity nail according to the invention, illustrated for illustrative and non-limiting purposes with the help of the attached drawing sheets in which:

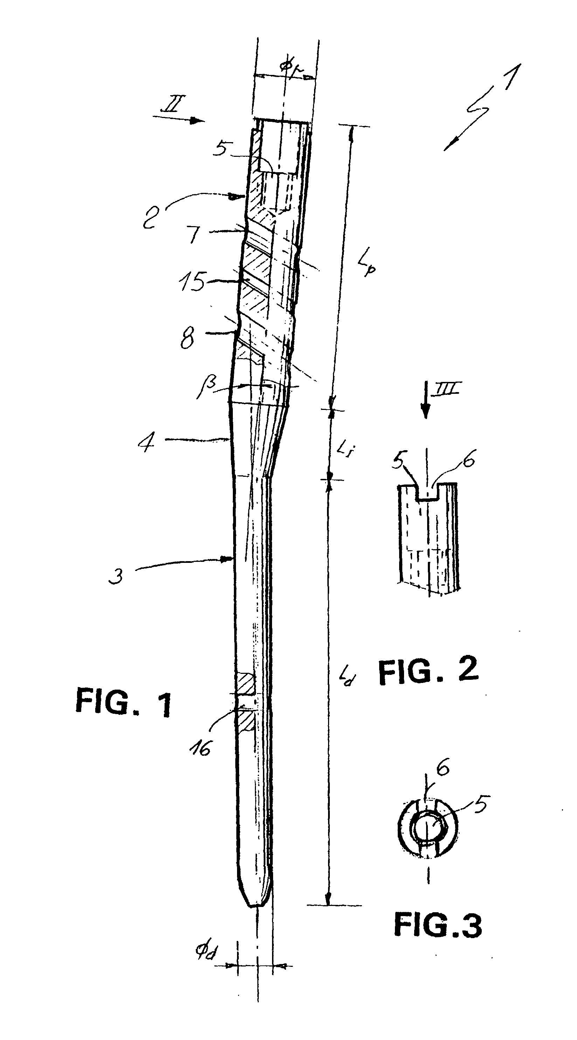

Fig. 1 illustrates a lateral elevation view of the intramedullay cavity nail according to a preferred aspect of the invention;
Fig. 2 illustrates a detail of the nail of Fig. 1 seen according to arrow II;
Fig. 3 illustrates a detail of Fig. 2 seen according to arrow III;

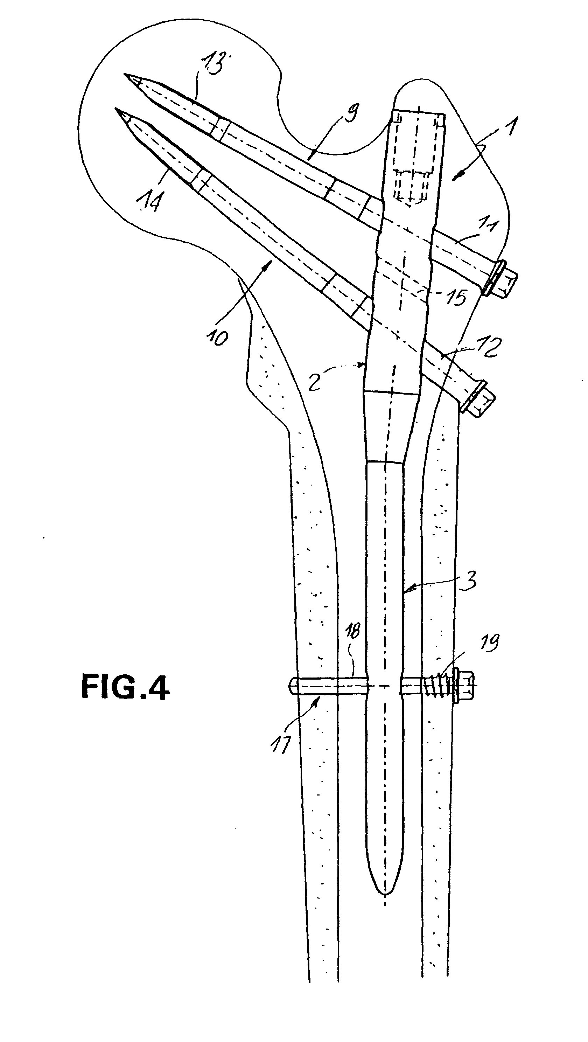

Figure 4 illustrates a lateral elevation view of the intramedullary cavity nail of Fig. 1 inserted in the medullary canal of a femur;
Figure 5 illustrates a lateral elevation view of a detail of the nail of Fig. 4;
Fig. 6 illustrates a lateral elevation view of another detail of the nail of Fig. 4;
Fig. 7 illustrates a lateral elevation view of a part of an instrument according to a preferred aspect of the invention for fixing the intramedullary cavity nail to the bone;

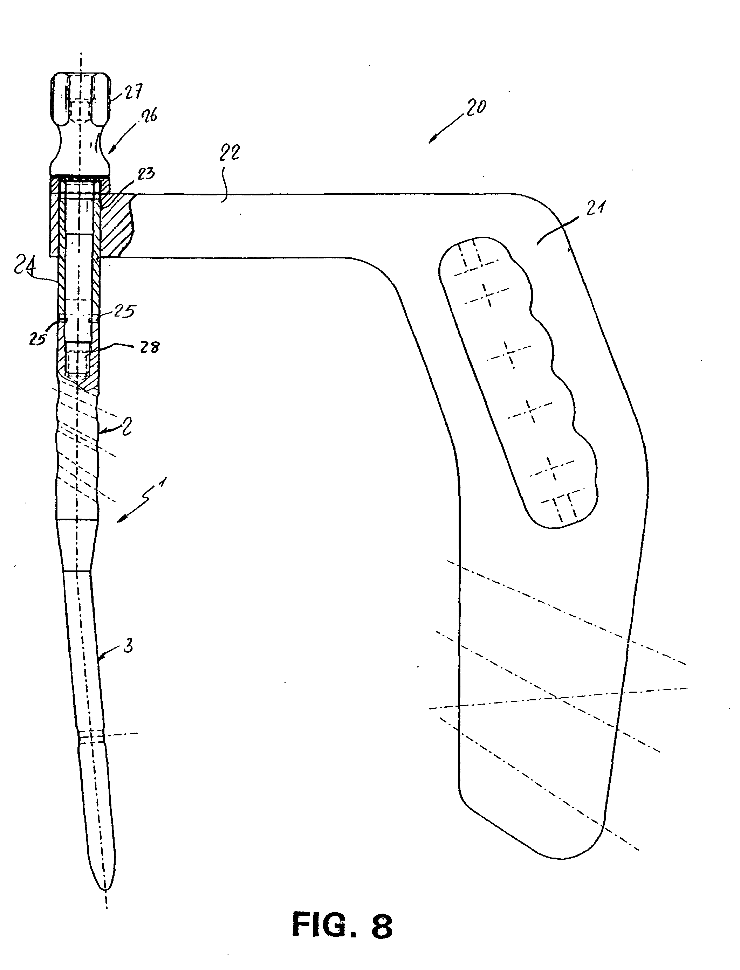

Fig. 8 illustrates a general lateral elevation view of the intramedullary cavity nail of Fig. 1, coupled to the instrument for insertion in the medullary canal.

### Best Modes for Carrying Out the Invention

With reference to the cited figures, an intramedullary cavity nail according to a preferred aspect of the invention, indicated globally by the reference numeral 1, is generally constituted by a solid elongated body in biocompatible metal, for example titanium or stainless steel of the type AISI 316LVM, essentially formed by a proximal portion 2 united to a distal portion 3 by means of a substantially truncated-conical intermediate portion 4.

Both the portions 2, 3 are substantially rectilinear and their axes form between them a deviation angle β of limited value, for example included between 4° and 5°.

The proximal portion 2, having a length Lₚ included for example between 70mm and 80mm, has a substantially constant and relatively high average diameter φₚ, for example comprised between 12mm and 16 mm and preferably equal to about 14mm, such as to be able to be stably anchored in a hole bored in the trochanteric zone of limited length of the bone. In this manner, one obtains a reduction of blood loss during the operation of tissue incision and a simplification of the surgical intervention with greater chances of success.

Towards the free end of the proximal portion 2, a threaded seat 5 is formed whose end edge has a diametrical notch 6.

In the proximal portion 2 there is provided at least one pair of transverse through holes 7, 8 for cephalic screws 9, 10 for stabilising the femoral head and inclined with respect to the axis aₚ of the proximal portion 2.

The holes 7, 8 are inclined with respect to the axis of the proximal portion 2 by an average angle of about 115° and may be mutually inclined by about 10° so as to be convergent, in order to prevent the shifting of the head and the exit of the screws therefrom.

Preferably, the cephalic screws 9, 10 have respective proximal end portions 11, 12 with possibly but not necessarily increased diameter, stably engageable in the holes 7, 8 of the nail 2, and distal end portions 13, 14 with lesser or equal diameter, and with self-threading tips for screwing into the femoral head.

If necessary, in the proximal portion 2 there may be provided a third through hole 15, inclined by an average angle of for example about 115° with respect to the axis aₚ for a medial Kirschner wire substantially coaxial to the femoral head, with a smaller diameter than the first two screws and not illustrated in the drawings.

The distal portion 3, having a length L_{d} included for example between 100mm and 120mm, has an average diameter φ_{d} less than diameter φₚ of the proximal portion, for example included between 8mm and 10mm and preferably equal to about 9mm, so as to be able to be easily inserted in the medullary canal of the femur without any boring of the same.

In the distal portion 3 a single through hole 16 is provided, substantially perpendicular to the axis a_{d}, for a diaphysis stabilisation and torsional blockage pin 17. The hole 16 is preferably provided at about half of the length L_{d} so as to result sufficiently distant from the free tip of the pin and reduce risks of breakage of its end portion.

Preferably, the diaphysis pin 17 has a smooth portion 18 with a substantially constant diameter stably engageable in the hole 15, and a threaded proximal end portion 19 engageable in the spongy tissue of the bone.

Thanks to this configuration, the nail 1 allows for an optimal stabilisation and a stable and quick synthesis of the bone with a hardly invasive technique and with limited blood loss.

Both the cephalic screws and the diaphysis pin may be provided with the same materials as the nail for obvious reasons of biocompatibility.

According to a further aspect of the invention, there is provided an instrument for positioning the above described intramedullary cavity nail and for performing the necessary drilling of the bone.

Advantageously, such instrument is constituted by a drilling mask 20 which extends in a principal plane and is formed by a grip 21 which is graspable by the surgeon and from which a transverse appendix 22 having an end hole 23 extends. A guide sleeve 24 is keyed in the hole 23 having diametrical protrusions or end teeth 25 at its lower end which pass through the principal plane of development of the mask and which are engageable in the diametrical notch 6 of the seat 5 of the nail 1 in order to guarantee its alignment during the intervention.

A pin 26 with a manoeuvre knob 27 may cross the internal cavity of the sleeve 24 and has a threaded end 28 which engages in the threaded seat 5 of the pin 1 in order to guarantee its connection with the mask 20.

In the lower part of the grip 21 of the mask 20 there are provided guide holes for bone drilling bits in correspondence with the holes 7, 8, 15 and 16 of the nail.

In use, the surgeon performs the drilling of the trochanteric zone of the femur for about 10cm, whereafter the nail is inserted taking care that the notch 6 along the edge of its threaded seat is positioned in a lateral plane of the limb. Thereafter the nail is connected to the drilling mask 20 taking care that the protrusions or teeth 25 of the guide sleeve 24 of the later are engaged in the notch 6. Finally, the pin 26 is inserted in the sleeve 24 and its threaded end 28 is screwed in the threaded seat 5 of the pin thereby providing its stable connection. At this point, the drilling of the bone may be initiated in correspondence with the holes 7, 8, 15, 16 of the pin and finally the screws 9, 10, 17 may be inserted in the respective holes for providing the stabilisation of the femur in a rapid and secure manner.

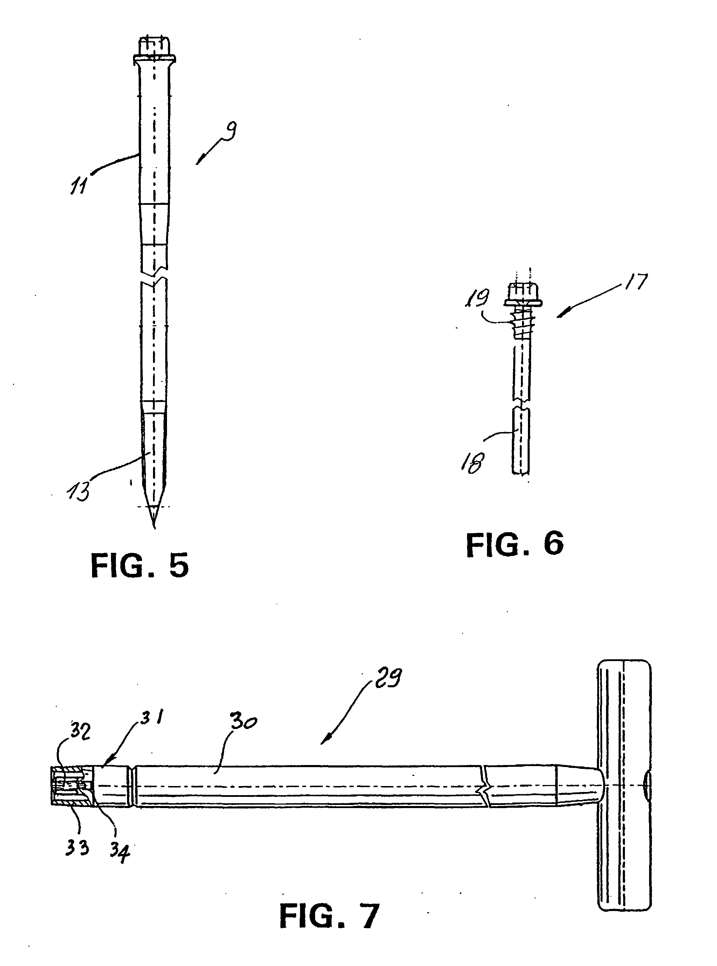

For the insertion and stabilisation of such screws while avoiding accidentally dropping them, it is possible to utilise a particular T-shaped tool, illustrated clearly in Fig. 7 and globally indicated by the reference numeral 29. The tool has a manoeuvring stem 30 with a head 31 having a central locking appendix 32 of hexagonal section engageable in the hexagonal fitting of the screw and an external tubular portion 33 with a diametrical longitudinal notch 34 for forming two semicylindrical elastic wings which grip the head of the screw.

## Claims

1. Intramedullary cavity nail for the treatment of femoral fractures, comprising a solid elongated body having a proximal portion (2) with at least one hole for accommodating corresponding screws (9,10) for stabilizing the femoral neck, joined to a distal portion (3) with at least one distal hole (16) for accommodating at least one diaphysis screw (17) for stabilizing the distal part of the femur, wherein said proximal portion (2) has a substantially constant diameter (Φₚ) adapted to be stably anchored in a relatively limited length bore of the femur for reducing blood losses, while said distal portion (3) has a substantially constant diameter (Φ_{d}) which is less than that of said proximal portion for being easily inserted in the medullary canal of the femur without any drilling, **characterized in that** said proximal portion (2) has a pair of holes (7, 8) adapted for accommodating respective cephalic screws (9, 10) for the fixing of the femoral head, said holes being inclined with respect to the axis (aₚ) of the proximal portion (2) and being also mutually inclined by about 10° so as to be convergent, in order to prevent the shifting of the femoral head and the exit of the screws therefrom.

2. Intramedullary cavity nail according to claim 1, **characterized in that** said holes (7, 8) in the proximal portion (2) are inclined with respect to the axis (aₚ) of the proximal portion by an average angle of about 115°.

3. Intramedullary cavity nail according to claim 1, **characterized in that** said cephalic screws (9, 10) have a notably smaller diameter than that of said proximal portion (2) and are slightly convergent with respect to one another and with respect to the median axis of the femoral head in order to exert on the latter a reaction force with a longitudinal component for contrasting its shifting and/or the axial penetration.

4. Intramedullary cavity nail according to claim 1, **characterized in that** said proximal and distal portions (2; 3) are substantially rectilinear and form between themselves a predetermined deviation angle (β) in a lateral plane.

5. Intramedullary cavity nail according to claim 4, **characterized in that** said angle of deviation (β) is comprised between 3° and 10° and is preferably equal to about 5°.

6. Intramedullary cavity nail according to claim 1, **characterized in that** the diameter (Φₚ) of said proximal portion (2) is comprised between 12 mm and 16mm and is preferably equal to about 14 mm.

7. Intramedullary cavity nail according to claim 1, **characterized in that** the diameter (Φ_{d}) of said distal portion (3) is comprised between 7 mm 25 and 11 mm and is preferably equal to about 9 mm.

8. Intramedullary cavity nail according to claim 2, **characterized in that** said cephalic screws (9, 10) have respective maximum diameter portions (11, 12) for the accommodation in corresponding holes (7, 8) of said proximal portion (2), with a maximum diameter comprised between 3 mm and 8 mm and preferably approximately 7mm.

9. Intramedullary cavity nail according to claim 1, **characterized in** of providing a single diaphysis screw (17) for contrasting torsional stresses of the nail, accommodated in a single hole (16) substantially perpendicular to the axis (a_{d}) of the distal portion (3) provided towards the end of the latter farther from said proximal portion (2).

10. Intramedullary cavity nail according to claim 9, **characterized in that** said diaphysis screw (17) has a substantially constant diameter principal portion (18) corresponding to that of said single hole (16) of said 20 distal portion, and an increased diameter threaded portion (19).

11. Intramedullary cavity nail according to claim 10, **characterized in that** said diaphysis screw (17) is positioned approximately at half length (Ld) of said distal portion for avoiding breakage of the nail towards its free end.

12. Intramedullary cavity nail according to claim 1, **characterized in that** said proximal portion (2) has, in correspondence with its free end, a threaded seat (5) for a connection pin (26) for connection to a drilling mask (20) arranged in the principal plane of the nail.

13. Intramedullary cavity nail according to claim 12, **characterized in that** the edge of said threaded seat (5) has a diametrical notch (6) coplanar with the principal plane of the nail for the insertion of alignment protrusions (25) between nail and mask (20).

14. Intramedullary cavity nail according to claim 12, characterized inthat said drilling mask (20) has a grip (21) with a transversal appendix (22) having at its free end an axial hole (23) for the passage of said connection pin (26).

15. Intramedullary cavity nail according to claim 14, **characterized in that** said grip (21) has a series of guide holes for drilling bits, alignable with the holes (7, 8, 15) of the cephalic and diaphysis screws in the drilling phase of the bone.

16. Intramedullary cavity nail according to claim 1, **characterized in that** said screws (9, 10, 17) have such heads to be engaged by a special tool (29), provided with a longitudinal stem (30) and a head (31) having a central locking appendix (32) and lateral gripping means (33) for engaging said screws heads ,for avoiding accidentally dropping them.

## Patentansprüche

1. Markraumnagel für die Behandlung von Frakturen des Femurs, umfassend einen festen länglichen Korpus mit einem proximalen Bereich (2) mit mindestens einem Loch zur Aufnahme korrespondierender Schrauben (9, 10) für die Stabilisierung des Femurhalses, der mit einem distalen Bereich (3) mit zumindest einem distalen Loch (16) zur Aufnahme zumindest einer Diaphysisschraube (17) für die Stabilisierung des distalen Bereiches des Femurs verbunden ist, wobei der proximale Bereich (2) einen im wesentlichen konstanten Durchmesser (Φₚ) aufweist, der für eine stabile Verankerung in einem Bohrloch des Femurs von relativ begrenzter Tiefe zur Reduzierung von Blutverlusten angepaßt ist, während der distale Bereich (3) einen im wesentlichen konstanten Durchmesser (Φ_{d}) aufweist, der geringer als der des proximalen Bereiches ist, um einfach ohne jegliches Bohren in den Markraumkanal des Femurs eingeführt zu werden, **dadurch gekennzeichnet, daß** der proximale Bereich (2) ein Paar von Löchern (7, 8) aufweist, die zur Aufnahme von jeweiligen Cephalicusschrauben (9, 10) für die Fixierung des Femurkopfes vorgesehen sind und die Löcher gegenüber der Achse (aₚ) des proximalen Bereiches (2) geneigt und auch wechselseitig um etwa 10° geneigt sind, so daß sie konvergent sind, um die Verlagerung des Femurkopfes und das Austreten der Schrauben aus diesem zu verhindern.

2. Markraumnagel nach Anspruch 1, **dadurch gekennzeichnet, daß** die Löcher (7, 8) im proximalen Bereich (2) gegenüber der Achse (aₚ) des proximalen Bereiches um einen durchschnittlichen Winkel von etwa 115° geneigt sind.

3. Markraumnagel nach Anspruch 1, **dadurch gekennzeichnet, daß** die Cephalicusschrauben (9, 10) einen merklich kleineren Durchmesser als den des proximalen Bereiches (2) aufweisen und in Bezug zueinander und gegenüber der Mittelachse des Femurkopfes leicht konvergent sind, um auf Letzeren eine Reaktionskraft mit einer Longitudinalkomponente auszuüben, die seiner Verlagerung und/oder der axialen Penetration entgegensteht.

4. Markraumnagel nach Anspruch 1, **dadurch gekennzeichnet, daß** der proximale und der distale Bereich (2; 3) im wesentlichen geradlinig sind und zwischen sich einen vorbestimmten Deviationswinkel (β) in einer lateralen Ebene ausbilden.

5. Markraumnagel nach Anspruch 4, **dadurch gekennzeichnet, daß** der Deviationswinkel (β) zwischen 3° und 10° enthalten ist und vorzugsweise gleich etwa 5° ist.

6. Markraumnagel nach Anspruch 1, **dadurch gekennzeichnet, daß** der Durchmesser (Φₚ) des proximalen Bereiches (2) zwischen 12 mm und 16 mm enthalten ist und vorzugsweise gleich etwa 14 mm ist.

7. Markraumnagel nach Anspruch 1, **dadurch gekennzeichnet, daß** der Durchmesser (Φ_{d}) des distalen Bereiches (3) zwischen 7 mm 25 und 11 mm enthalten ist und vorzugsweise gleich etwa 9 mm ist.

8. Markraumnagel nach Anspruch 2, **dadurch gekennzeichnet, daß** die Cephalicusschrauben (9, 10) jeweils Maxiamldurchmesserbereiche (11, 12) zur Aufnahme in entsprechenden Löchern (7, 8) des proximalen Bereiches (2) aufweisen, wobei der Maximaldurchmesser zwischen 3 und 8 mm enthalten und vorzugsweise angenähert 7 mm ist.

9. Markraumnagel nach Anspruch 1, **dadurch gekennzeichnet, daß** eine einzige Diaphysisschraube (17) zur Entgegenwirkung von Torsionsbelastungen des Nagels vorgesehen ist, welche in einem einzelnen Loch (16), das im wesentlichen rechtwinklig zur Achse (a_{d}) des distalen Bereiches (3) und in Richtung auf das weiter von dem proximalen Bereich (2) entfernt liegende Ende vorgesehen ist, aufgenommen wird.

10. Markraumnagel nach Anspruch 9, **dadurch gekennzeichnet, daß** die Diaphysisschraube (17) einen Hauptabschnitt (18) mit im wesentlichen konstantem Durchmesser, der mit dem des einzelnen Loches (16) des distalen Bereiches korrespondiert, und einen Gewindeabschnitt (19) vergrößerten Durchmessers aufweist.

11. Markraumnagel nach Anspruch 10, **dadurch gekennzeichnet, daß** die Diaphysisschraube (17) annähernd auf der halben Länge (Ld) des distalen Bereiches positioniert ist, um einen Bruch des Nagels an seinem freien Ende zu vermeiden.

12. Markraumnagel nach Anspruch 1, **dadurch gekennzeichnet, daß** der proximale Bereich (2) einen mit seinem freien Ende korrespondierenden Gewindesitz (5) für einen Verbindungszapfen (26) zur Verbindung mit einer in der Hauptebene des Nagels angeordneten Bohrschablone (20) aufweist.

13. Markraumnagel nach Anspruch 12, **dadurch gekennzeichnet, daß** der Rand des Gewindesitzes (5) eine diametale Nut (6) coplanar zur Hauptebene des Nagels für die Einführung von Ausrichtungsvorsprüngen (25) zwischen Nagel und Schablone (20) aufweist.

14. Markraumnagel nach Anspruch 12, **dadurch gekennzeichnet, daß** die Bohrschablone (20) einen Griff (21) mit einem quer verlaufenden Ansatz (20) mit einem axialen Loch (23) zum Durchtritt des Verbindungszapfens (26) an seinem freien Ende aufweist.

15. Markraumnagel nach Anspruch 14, **dadurch gekennzeichnet, daß** der Griff (21) eine Reihe von Führungslöchern für Bohrerspitzen aufweist, die nach den Löchern (7, 8, 15) der Cephalicus- und Diaphysisschrauben in der Bohrphase des Knochens ausrichtbar sind.

16. Markraumnagel nach Anspruch 1, **dadurch gekennzeichnet, daß** die Schrauben (9, 10, 17) solche von einem Spezialwerkzeug (29) betätigbare Köpfe aufweisen, das mit einem länglichen Stiel (30) und einem Kopf (31) mit einem zentralen Verriegelungsansatz (32) und quer verlaufenden Greifmitteln (33) zum Verriegeln der Schraubenköpfe ausgebildet ist, um ein versehentliches Herabfallen derselben zu vermeiden.

## Revendications

1. Clou de cavité intramédullaire pour le traitement des fractures fémorales, comprenant un corps allongé massif possédant une partie proximale (2) avec au moins un trou pour recevoir des vis correspondantes (9, 10) pour stabiliser le col fémoral, relié à une partie distale (3) avec au moins un trou distal (16) pour recevoir au moins une vis de diaphyse (17) pour stabiliser la partie distale du fémur, où ladite partie proximale (2) a un diamètre substantiellement constant (Φₚ) adapté pour être ancré de manière stable dans un perçage de longueur limitée du fémur pour réduire les pertes sanguines, tandis que ladite partie distale (3) possède un diamètre substantiellement constant (Φ_{d}) qui est inférieur à celui de ladite partie proximale pour être aisément inséré dans le canal médullaire du fémur sans aucun perçage, **caractérisé en ce que** ladite partie proximale (2) possède deux trous (7, 8) adaptés pour recevoir des vis céphaliques respectives (9, 10) pour la fixation de la tête fémorale, lesdits alésages étant inclinés par rapport à l'axe (aₚ) de la partie proximale (2) et étant également mutuellement inclinés d'environ 10° de manière à être convergents, afin d'empêcher que la tête fémorale ne se décale et que les vis n'en sortent.

2. Clou de cavité intramédullaire selon la revendication 1, **caractérisé en ce que** lesdits trous (7, 8) dans la partie proximale (2) sont inclinés par rapport à l'axe (aₚ) de la partie proximale d'un angle moyen d'environ 115°.

3. Clou de cavité intramédullaire selon la revendication 1, **caractérisé en ce que** lesdites vis céphaliques (9, 10) possèdent un diamètre notablement inférieur à celui de ladite partie proximale (2) et sont légèrement convergents l'un par rapport à l'autre et par rapport à l'axe médian de la tête fémorale afin d'exercer sur cette dernière une force de réaction avec une composante longitudinale s'opposant à son décalage et/ou à la pénétration axiale.

4. Clou de cavité intramédullaire selon la revendication 1, **caractérisé en ce que** lesdites parties proximale et distale (2 ; 3) sont substantiellement rectilignes et forment entre elles un angle de déviation prédéterminé (β) dans un plan latéral.

5. Clou de cavité intramédullaire selon la revendication 4, **caractérisé en ce que** ledit angle de déviation (β) est compris entre 3° et 10° et est de préférence égal à environ 5°.

6. Clou de cavité intramédullaire selon la revendication 1, **caractérisé en ce que** le diamètre (Φₚ) de ladite partie proximale (2) est compris entre 12 mm et 16 mm et est de préférence égal à environ 14 mm.

7. Clou de cavité intramédullaire selon la revendication 1, **caractérisé en ce que** le diamètre (Φ_{d}) de ladite partie distale (3) est compris entre 7 mm 25 et 11 mm et est de préférence égal à environ 9 mm.

8. Clou de cavité intramédullaire selon la revendication 2, **caractérisé en ce que** lesdites vis céphaliques (9, 10) possèdent des parties de diamètre maximum (11, 12) pour être reçues dans des trous correspondants (7, 8) de ladite partie proximale (2), avec un diamètre maximum compris entre 3 mm et 8 mm et de préférence d'approximativement 7 mm.

9. Clou de cavité intramédullaire selon la revendication 1, **caractérisé en ce qu'**il est prévu une unique vis de diaphyse (17) pour s'opposer aux contraintes de torsion du clou, reçue dans un trou unique (16) substantiellement perpendiculaire à l'axe (a_{d}) de la partie distale (3), formé en allant vers l'extrémité de cette dernière plus loin que ladite partie proximale (2).

10. Clou de cavité intramédullaire selon la revendication 9, **caractérisé en ce que** ladite vis de diaphyse (17) possède une partie principale (18) de diamètre sensiblement constant correspondant à celui dudit trou unique (16) de ladite partie distale, et une partie filetée (19) de diamètre accru.

11. Clou de cavité intramédullaire selon la revendication 10, **caractérisé en ce que** ladite vis de diaphyse (17) est positionnée approximativement à mi-distance (Ld) de ladite partie distale pour évite la cassure du clou en direction de son extrémité libre.

12. Clou de cavité intramédullaire selon la revendication 1, **caractérisé en ce que** ladite partie proximale (2) possède, en correspondance avec son extrémité libre, un siège fileté (5) pour une broche de liaison (26) pour liaison à un masque de perçage (20) disposé dans le plan principal du clou.

13. Clou de cavité intramédullaire selon la revendication 12, **caractérisé en ce que** le bord dudit siège fileté possède un cran diamétral (6) coplanaire avec le plan principal du clou pour l'insertion de saillies d'alignement (25) entre le clou et le masque (20).

14. Clou de cavité intramédullaire selon la revendication 12, **caractérisé en ce que** ledit masque de perçage (20) possède un manche (21) avec un appendice transversal (22) possédant à son extrémité libre un trou axial (23) pour le passage de ladite broche de liaison (26).

15. Clou de cavité intramédullaire selon la revendication 14, **caractérisé en ce que** ledit manche (21) possède une série de trous de guidage pour des forets de perçage, alignables sur les trous (7, 8, 15) des vis céphaliques et de diaphyse lors de la phase de perçage de l'os.

16. Clou de cavité intramédullaire selon la revendication 1, **caractérisé en ce que** lesdites vis (9, 10, 17) ont des têtes telles qu'elles viennent en prise avec un outil spécial (29), pourvu d'une tige longitudinale (30) et d'une tête (31) possédant un appendice de verrouillage central (32) et des moyens de saisie latéraux (33) pour venir en prise avec lesdites têtes de vis, pour éviter de les laisser tomber accidentellement.
